# EUROPEAN PATENT SPECIFICATION

(11) **EP 3 445 063 B1**
(45) Date of publication and mention of the grant of the patent: **22.04.2020**
(21) Application number: 18189258.9
(22) Date of filing: 16.08.2018
(51) Int. Cl.: H04R 1/10, G08B 21/18

(54) **SYSTEM AND METHOD FOR HEARING PROTECTION DEVICE TO COMMUNICATE ALERTS FROM PERSONAL PROTECTION EQUIPMENT TO USER**
SYSTEM UND VERFAHREN FÜR EINE GEHÖRSCHUTZVORRICHTUNG ZUR ÜBERMITTLUNG VON ALARMEN VON PERSÖNLICHEN SCHUTZAUSRÜSTUNGEN AN DEN BENUTZER
SYSTÈME ET PROCÉDÉ POUR DISPOSITIF DE PROTECTION AUDITIVE PERMETTANT DE COMMUNIQUER DES ALERTES D'UN ÉQUIPEMENT DE PROTECTION PERSONNELLE À UN UTILISATEUR

(30) Priority: 18.08.2017 IN 201711029320
(43) Date of publication of application: 20.02.2019
(73) Proprietor: Honeywell International Inc., Morris Plains, NJ New Jersey 07950 (US)
(72) Inventor: RACHAKONDA, Nagaraju, Morris Plains, NJ 07950 (US); KANAJALA, LG Srinivasa Rao, Morris Plains, NJ 07950 (US); SHAIK, Mehabube Rabbanee, Morris Plains, NJ 07950 (US)
(74) Representative: Haseltine Lake Kempner LLP

(56) References cited:
- WO-A1-2016/089708
- WO-A1-2017/030837

## Description

### CROSS-REFERENCE TO RELATED APPLICATIONS

The present application claims priority to India Provisional Patent Application No. 201711029320 filed August 18, 2017 by Nagaraju Rachakonda, et al. and entitled "System and Method for Hearing Protection Device to Communicate Alerts from Personal Protection Equipment to User".

### STATEMENT REGARDING FEDERALLY SPONSORED

### RESEARCH OR DEVELOPMENT

Not applicable.

### REFERENCE TO A MICROFICHE APPENDIX

Not applicable.

### BACKGROUND

While working in an environment with high noise levels, a user may wear a headset that comprises noise reduction or cancellation devices. In some cases, a user may also wear personal protection equipment (PPE) configured to generate alerts and/or alarms while working in hazardous environments, such as a gas detector, a self-contained breathing apparatus (SCBA), a powered air purifying respirator (PAPR), and/or other protection equipment or environmental sensors.

WO2017030837A1 discloses a communication headset, which may comprise active noise cancellation or reduction, configured to wirelessly communicate with one or more PPE devices using voice recognition to process voice inputs from a user. The headset may comprise a voice recognition module configured to receive voice inputs from a user (via a microphone on the headset) and convert the voice inputs to text. The voice inputs may comprise commands that may be sent to the PPE devices, wherein the commands may request information from the PPE devices, such as pressure level, temperature, gas content and level, battery life, etc., and wherein the PPE devices may send a response to the headset comprising that information. The headset may then convert the response to a voice output, which may then be sent to the user via speakers in the headset.

WO2016089708A1 discloses systems and methods for communication between one or more personal protection equipment (PPE) devices, a mobile device, and a central monitoring station. Personnel may wear PPE devices for detection and communication. These portable devices may communicate wirelessly, over a wireless fidelity (Wi-Fi) network, via Bluetooth, or another wireless connection. Systems may include a smartphone application operable to receive and combine information from each of the PPE devices. The application may also display the information to the user. In some embodiments, the application may transfer the data to a cloud storage network via a cellular network. Additionally, the application may communicate the combined data from all of the PPE devices to the central monitoring station.

### SUMMARY

The present invention in its various aspects is as set out in the appended claims. In an embodiment, a wireless communication system may comprise at least one personal protection equipment device configured to generate messages and wirelessly communicate the generated messages; and a hearing protection headset comprising passive noise cancellation material configured to protect a user from harmful sound exposure; a wireless communication module configured to wirelessly receive one or more messages from the at least one personal protection equipment device, and convert the received message to speech format; and one or more speakers configured to communicate the converted message to the user.

In an embodiment, a method for communicating data from a personal protection equipment device to a headset may comprise generating at least one message, by a personal protection equipment device, comprising information related to the personal protection equipment device; wirelessly communicating the generated message from the personal protection equipment device; receiving, by a hearing protection headset, one or more of the generated messages from the at least one personal protection equipment device; converting, by the hearing protection headset, the received message to speech format; and communicating, via one or more speakers within the hearing protection headset, the converted message to the user.

In an embodiment, a hearing protection headset may comprise passive noise cancellation material configured to protect a user from harmful sound exposure; a wireless communication module configured to wirelessly receive one or more data messages generated by at least one personal protection equipment device; a converter configured to convert the received message to speech format; and one or more speaker configured to communicate the converted message to the user.

### BRIEF DESCRIPTION OF THE DRAWINGS

For a more complete understanding of the present disclosure, reference is now made to the following brief description, taken in connection with the accompanying drawings and detailed description, wherein like reference numerals represent like parts.
FIG. 1 illustrates a wireless communication system comprising a plurality of PPE devices according to an embodiment of the disclosure.
FIG. 2 illustrates another view of the communication system comprising a headset and a plurality of PPE devices according to an embodiment of the disclosure.
FIG. 3 illustrates a diagram of an electronics system of a headset according to an embodiment of the disclosure.
FIGS. 4A-4B illustrate perspective and exploded views of a headset according to an embodiment of the disclosure.

### DETAILED DESCRIPTION

Embodiments of the disclosure include systems and methods for wirelessly communicating messages from one or more PPE devices to a hearing protection headset, and communicating the messages to a user via one or more speakers of the hearing protection headset.

Typically, hearing protection personal protection equipment (PPE) (such as earmuffs or earplugs) may be used in noisy environments to limit the sound exposure to a worker. Along with hearing PPE, a worker may also wear and/or carry other PPE, such as gas detectors, respiratory protection, and man-down detection. Electronic PPE typically generate device alerts and critical information using audio alerts, visual alerts, wireless messages, etc. If a worker is wearing hearing protection, which is required in some work environments, these alerts from PPE may not be noticed by the worker, especially in a noisy environment, which could endanger the worker and others around them.

Some hearing protection PPE may generate local device alerts, such as sound exposure level, battery level, and other critical alerts (e.g. related to the hearing protection PPE/earmuffs/headset). However, to better enable a user to receive alerts and notifications from other PPE, the hearing protection earmuffs may be interfaced with the other PPE (carried by or worn by or located in proximity to the user) to collect the critical data generated by the PPE and deliver alerts to the user in a reliable fashion. The hearing protection earmuffs may also be used as a gateway for further communication of the PPE data, such as to cloud storage, central monitoring, and/or similar destinations.

The hearing protection earmuffs may be enabled with wireless communication (such as Bluetooth, and/or Wi-Fi) and a common protocol may be defined to collect data from other PPE devices. The other PPE devices may be enabled with the same wireless communication protocol (and include wireless communication means, such as a transmitter or transceiver), may broadcast critical alerts and/or other PPE data that is to be received by the hearing protection earmuffs. In some embodiments, the data that is broadcast by the PPE devices may be generated by the PPE devices in text format (which will be received by the headset/earmuffs, converted to speech or audio format, and communicated to the user as a voice alert, e.g. by speakers in the earmuffs/headset directed into the user's ear canal) and/or binary format (which can be communicated to a processor for the headset/earmuff (which might then use that binary data to generate an alert via the speakers, for example) and/or other devices, such as a central computer or cloud computing system, for example via a gateway). In some alternate embodiments, (for example, where the other PPE are not configured to transmit alerts via text, but perhaps use an audio or visual alert), the hearing protection earmuff/headset might include a sensor configured (e.g. of the relevant type and oriented towards the other PPE's alert output) to detect the alert (e.g. audio and/or visual) from the other (e.g. non-hearing protection) PPE device(s).

In some embodiments, the headset/earmuff may also comprise (non-volatile) memory, for example with a look-up table correlating received alerts with preloaded messages and/or voice prompts (for example which might then be transmitted to the speakers and thereby directed into the user's ear canal). And in some embodiments, if multiple alerts are received by the earmuffs/headset in close proximity (e.g. simultaneously or substantially simultaneously), the processor of the headset/earmuff may queue the information (e.g. for display into the ear via speaker and/or transmittal to other devices, for example by gateway) based on criticality (e.g. with the most critical information (as pre-selected and set, for example in memory) being displayed and/or transmitted first). In some embodiments, the criticality may be determined by a PPE device before the alert is sent to the headset. In some embodiments, the criticality may be determined by the headset.

In some embodiments, the headset may not comprise active noise cancellation. In some embodiments, the headset may not comprise a user interface to control or configure the communication between the headset and the PPE device(s). In some embodiments, the headset may not be configured to recognize which PPE device has sent a data message. In some embodiments, a user or wearer of the headset may not request the data messages from the PPE device, the headset will only receive messages generated and broadcast by the PPE device. In some embodiments, the headset may not comprise a microphone nor a voice recognition system. In some embodiments, the headset and the PPE devices may communicate directly with one another without a mobile device, communicator device, or any other device facilitating the communication between the headset and the PPE devices.

This may reduce the number and complexity of the elements required to be in the headset to allow the communication between the PPE devices and the headset, wherein the headset may be configured to receive information from the PPE devices without a complex communication between the PPE devices and the headset. Additionally, the reduced complexity of the headset may result in a cheaper or inexpensive headset, which may therefore appeal to a broader range of consumers, including less sophisticated consumers. Therefore the simplicity of the headset, and the simplified set-up, pairing, and communication between the PPE and the headset may allow a less sophisticated consumer to easily operate the headset and the communication from the PPE devices.

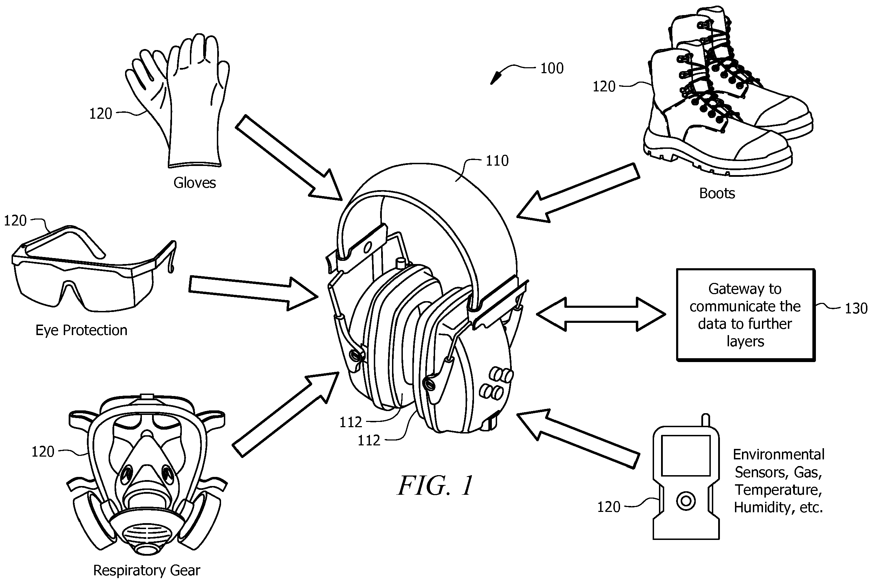

Referring now to FIG. 1, a communication system 100 is shown, wherein the system 100 comprises a headset 110 (which may also be called earmuffs, hearing protection, PPE, or something similar), where the headset 110 may be configured to receive data from one or more PPE devices 120. The PPE devices 120 may be located proximate to the headset 110, for example, when they are worn and/or carried by the user wearing the headset 110. The PPE devices 120 may be configured to generate data messages, and wirelessly broadcast the data messages. The headset 110 may receive the data messages, and may convert the data messages to speech (e.g. via a processor), which may then be broadcast to the user via one or more speakers located within the headset 110. In some embodiments, the headset 110 may be configured to communicate (e.g. wirelessly) with a gateway 130, where the headset 110 may forward data received from the PPE devices 120 via the gateway 130 to other devices and/or storage systems. In some embodiments, the gateway 130 may be part of (e.g. integral with) the headset/earmuff 110 (e.g. the processor of the earmuff 110 may include a transceiver configured to forward received data), while in other embodiments the headset 110 may not include a transmitter/transceiver with sufficient range, but may instead communicate with an alternate gateway device (e.g. a smartphone configured to interact with the earmuff and with other devices, thereby serving as a conduit for longer-range communication). The PPE devices 120 may include, but are not limited to: gloves, eye protection, respiratory protection or gear, boots, and/or environmental sensors (such as temperature sensors, pressure sensors, gas sensors, humidity sensors, etc.).

In some embodiments, the headset 110 may comprise passive noise cancellation, such as in the form of noise cancellation material 112 in the earmuffs of the headset 110. In some embodiments, the headset 110 may not be configured to send any messages to the PPE devices 120. In other words, the communication between the headset 110 and the PPE device 120 may be one-way from the PPE devices 120 to the headset. This may reduce the number and complexity of the elements required to be in the headset 110 to allow the communication between the PPE devices 120 and the headset 110, wherein the headset 110 may be configured to receive information from the PPE devices 120 without a complex communication between the PPE devices 120 and the headset 110. Additionally, the headset 110 may not be configured to control any of the PPE devices 120. In some embodiments, the headset 110 may not recognize the PPE devices 120, but may only receive broadcast messages; while in other embodiments, the headset may pair to the other relevant PPE so that it may recognize the source of the messages/received transmissions.

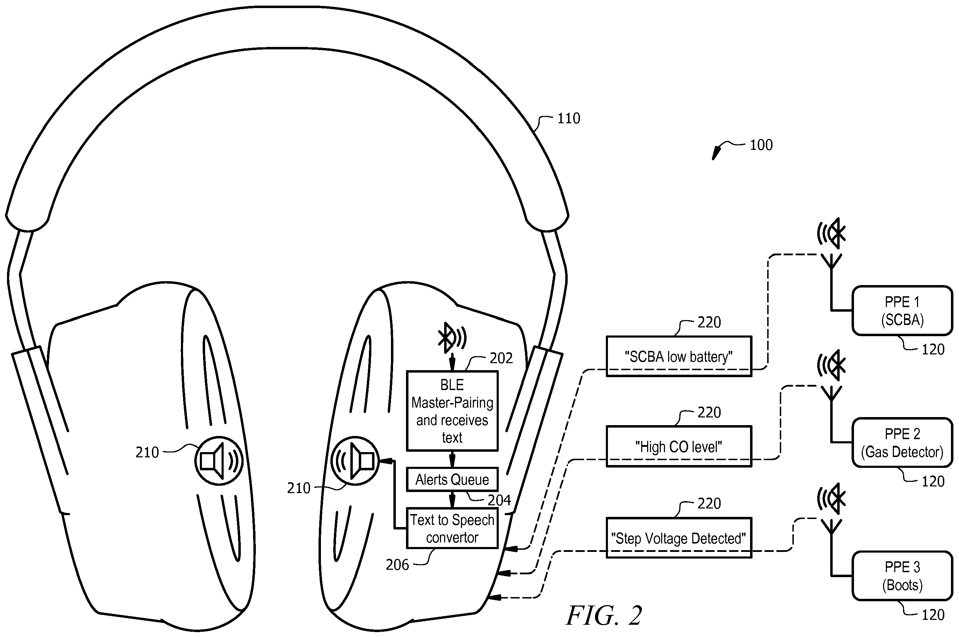

Referring to FIG. 2, another view of the communication system 100 is shown. The PPE devices 120 may generate data messages 220 that may be broadcast by the PPE devices 120. The headset 110 may comprise a wireless interface 202 configured to receive the data messages 220. The headset 110 may comprise a memory 204 configured to at least temporarily store (and possibly queue) the received data messages 220. In some embodiments, the PPE devices 120 may be configured to evaluate and prioritize the data messages 220 before sending to the headset 110. The headset 110 may be configured to rank the plurality of received data messages 220 based on a priority level of each message, and queue the messages in a memory (and/or an alerts queue) 204 to be sent to the speakers 210. In some embodiments, each of the PPE devices 120 may be associated with a priority level (e.g., relative to one another and/or independently), and the received data messages 220 may be ranked based on the priority level of the PPE device 120 that generated the message 220.

In some embodiments, the PPE devices 120 may be configured to determine a priority level for each data message 220 generated by the PPE device 120 based on preset priority levels, which may be stored in a memory of the PPE devices 120 and/or may be accessed by the PPE devices 120. The headset 110 may be configured to recognize the priority level for each data messages 220 received by the headset 110. The priority level may be determined based on the information that is included in the data messages 220. For example, the PPE device 120 may be configured to generate multiple types of messages that may contain a range of information, where the information may be informative, critical, and/or other levels of criticality. Certain PPE devices 120 may generate alarms (e.g., gas detection alarms, temperature level alarms, respiratory level alarms, etc.) that may be deemed critical, and therefore high priority, information. The same PPE devices 120 may also generate low level alerts (such as battery level alerts, lower level sensor alerts, maintenance alerts, etc.) that may be deemed informative, and therefore lower priority. The PPE devices 120 may also generate any range of criticality of alerts, where the priority levels may comprise at least two levels, and may in some embodiments comprise more than two levels. The headset 110 may receive and communicate all of the data messages 220 generated by the PPE devices 120, but higher priority messages (e.g., alarms) may be ranked and queued above lower priority messages (e.g., alerts).

The headset 110 may comprise a converter 206 configured to convert the received messages to audio (or speech), and configured to send the converted messages to one or more speakers 210 in the headset 110. The converter 206 may be configured to process messages in text format and convert them to speech format to be played by the speakers 210. The converter 206 may be configured to processing messages in binary format and convert them to speech format to be played by the speakers 210. In some embodiments, the data messages 220 may be ranked and queued in the memory 204 before they are processed by a text to speech converter 206. In other embodiments, the alerts may be processed by the converter 206 before they are ranked and queued.

In some embodiments, each of the one or more PPE 120 may comprise one or more processors, at least one memory, and an application stored in the memory and executed by the processor that is configured to collect data associated with the PPE 120, process the data to determine if a message should be sent to the headset, generate the one or more data messages 220, and associate the one or more data messages 220 with a priority level. The priority level may be chosen from a preset list of priority levels, and the preset list of priority levels may be common across a plurality of PPE devices 120 (in some embodiments, all of the PPE devices 120 communicating with the headset 110).

In the embodiment shown in FIG. 2, the system 100 may communicate wirelessly using Bluetooth protocol. In some embodiments, each of the PPE devices 120 may comprise a near-field communication (NFC) module and the headset 110 may comprise an NFC module configured to interact to initiate a Bluetooth pairing between the headset 110 and the PPE devices 120. In other words, NFC between the two devices (when they are in close proximity), may act to link the two devices and to automatically activate Bluetooth transmission of data from the PPE devices 120 to the now linked headset 110. When the PPE device 120 is placed in proximity (e.g. within NFC range, such as within 10 cm or within 3 cm) to the headset 110, a NFC peer-to-peer (P2P) data exchange may occur between the headset 110 and the PPE device(s) 120, where the data exchange may include identification and connection data associated with the PPE device 120 and/or headset 110.

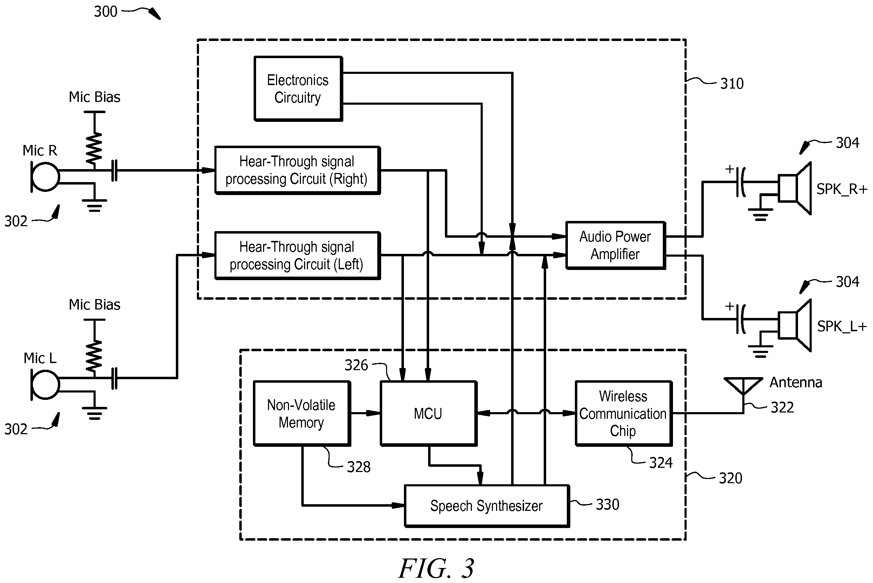

Referring to FIG. 3, a diagram of an electronics system 300 is shown, wherein the electronics system 300 may be located within a headset 110 (described above). The electronics system 300 may comprise one or more microphones 302 and one or more speakers 304. The electronics system 300 may also comprise additional circuitry 310 configured to receive, process, and communicate data between the microphones 302 and the speakers 304.

Additionally, the electronics system 300 may comprise a wireless communication module 320 configured to receive, process, and communicate data from outside devices (e.g., PPE devices described above) to the headset 110. The wireless communication module 320 may comprise a wireless antenna 322 and a wireless communication chip 324 configured to receive and possibly transmit wireless data. The wireless communication module 320 may comprise a processor (or microcontroller unit (MCU)) 326 and a memory 328. The wireless communication module 320 may also comprise a speech synthesizer (or converter) 330 configured to receive data via the wireless communication chip 324, and convert the received data to speech (or audio) format. The converted data may then be forwarded to the one or more speakers 304 to be heard by the user.

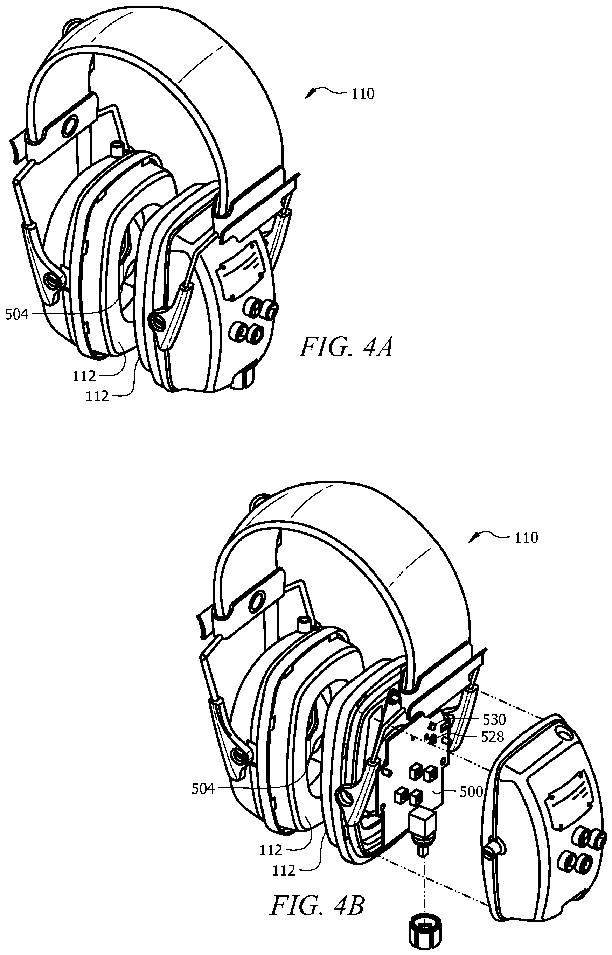

FIGS. 4A-4B illustrate detailed views of an exemplary embodiment of a headset 110, where the headset 110 may comprise an electronics system 500 configured to receive and process messages from one or more PPE devices, as described above. The electronics system 500 may comprise a speech synthesizer (or converter) 530, as described above, and a non-volatile memory 528, as described above, which may comprise pre-loaded voice prompts. The pre-loaded or pre-set voice prompts may be associated with expected information to be received from one or more PPE devices. The electronics system 500 may comprise and/or communicate with one or more speakers 504 within the headset 110.

In an embodiment, a wireless communication system may comprise at least one personal protection equipment (PPE) device configured to generate messages and wirelessly communicate the generated messages; and a hearing protection headset comprising passive noise cancellation material configured to protect a user from harmful sound exposure; a wireless communication module configured to wirelessly receive one or more messages from the at least one PPE device, and convert the received message to speech (or audio) format; and one or more speakers configured to communicate the converted message to the user.

## Claims

1. A wireless communication system (100) comprising:
at least one personal protection equipment device (120) configured to:
generate a plurality of messages and wirelessly communicate the plurality of generated messages; and
determine a priority level for the plurality of generated messages based on a preset list of priority levels;
a hearing protection headset (110) comprising:
passive noise cancellation material (112) configured to protect a user from harmful sound exposure;
at least one processor (326) configured to:
receive a plurality of messages from one or more personal protection equipment device (120);
rank the plurality of messages based on the determined priority level; and
queue the plurality of messages to be communicated to the user in the ranked order; and
convert the queue of the plurality of messages to speech format; and
one or more speaker (210) configured to communicate the converted plurality of messages to the user.

2. The wireless communication system (100) of claim 1, wherein the at least one personal protection equipment device (120) comprises a plurality of personal protection equipment devices (120), and wherein each of the plurality of personal protection equipment devices (120) is associated with a priority level.

3. The wireless communication system (100) of claim 2, wherein the at least one processor (326) is further configured to:
rank the plurality of messages based on the priority level of the personal protection equipment devices (120) associated with the messages.

4. The wireless communication system (100) of claim 1, further comprising a wireless communication module (320) of the headset (110), wherein the wireless communication module (320) comprises a near-field communication module, and wherein each of the at least one personal protection equipment device (120) comprises a near-field communication module.

5. The wireless communication system (100) of claim 4, wherein, when the personal protection equipment device (120) is in proximity to the headset (110) within near-field communication range, wherein the at least one processor (326) configured to:
complete a near-field communication peer-to-peer data exchange between the headset (110) and the personal protection equipment device (120); and
automatically activate a Bluetooth connection between the headset (110) and the personal protection equipment device (120) via the peer-to-peer data exchange.

6. The wireless communication system (100) of any of claims 1 to 5, wherein the wireless communication module (320) of the headset (110) comprises a Bluetooth communication module; and a text to speech converter.

7. A method for communicating data from a personal protection equipment device (120) to a headset (110) comprising:
generating a plurality of messages, by a personal protection equipment device (120), comprising information related to the personal protection equipment device (120);
determining a priority level for the plurality of generated messages based on a preset list of priority levels, wherein the preset list of priority levels is common across a plurality of personal protection equipment devices(s) (120);
wirelessly communicating the plurality of generated messages from the personal protection equipment device (120);
receiving, by the hearing protection headset (110), a plurality of messages from one or more personal protection equipment device (120);
ranking, by the hearing protection headset (110), the plurality of messages based on the determined priority level; and
queuing, by the hearing protection headset (110), the plurality of messages to be communicated to the user in the ranked order.
converting, by the hearing protection headset (110), the queue of the plurality of messages to speech format; and
communicating, via one or more speaker (210) within the hearing protection headset (110), the plurality of converted messages to the user.

8. The method of claim 7, wherein determining the priority level is performed by the personal protection equipment device (120).

9. The method of any of claims 7 or 8, further comprising:
determining a priority level for each of the plurality of personal protection equipment devices (120);
ranking the plurality of messages based on the determined priority level of the personal protection equipment devices (120) associated with the messages; and

10. The method of any of claims 7 to 9, further comprising protecting a user from harmful sound exposure via passive noise cancellation material (112) within the hearing protection headset (110).

11. The method of any of claims 7 to 10, further comprising:
placing the personal protection equipment device (120) in proximity to the headset (110) within near-field communication range;
completing a near-field communication peer-to-peer data exchange between the headset (110) and the personal protection equipment device (120); and
automatically activating a Bluetooth connection between the headset (110) and the personal protection equipment device (120) via the peer-to-peer data exchange.

12. The method of claim 11, wherein wirelessly communicating the plurality of generated messages from the personal protection equipment device (120), and receiving the plurality of generated messages by the hearing protection headset (110) is completed over the Bluetooth.

13. The wireless communication system (100) of claim 1, wherein the priority level comprises a first level of criticality and a second level of criticality, and
wherein the first level of criticality corresponds to at least one of gas detection alarms, temperature level alarms, respiratory level alarms, and the second level of criticality corresponds to at least one of battery level alerts, lower level sensor alerts, and maintenance alerts.

## Patentansprüche

1. Drahtloses Kommunikationssystem (100), umfassend:
mindestens eine persönliche Schutzausrüstungsvorrichtung (120), konfiguriert zum:
Erzeugen einer Vielzahl von Nachrichten und drahtlosen Übermitteln der Vielzahl von erzeugten Nachrichten; und
Bestimmen einer Prioritätsstufe für die Vielzahl von erzeugten Nachrichten basierend auf einer voreingestellten Liste von Prioritätsstufen;
ein Headset mit Gehörschutz (110), umfassend:
Material zur passiven Geräuschunterdrückung (112), das konfiguriert ist, um einen Benutzer vor schädlicher Schallbelastung zu schützen;
mindestens einen Prozessor (326), konfiguriert zum:
Empfangen einer Vielzahl von Nachrichten von einer oder mehreren persönlichen Schutzausrüstungsvorrichtungen (120);
Reihen der Vielzahl von Nachrichten basierend auf der bestimmten Prioritätsstufe; und
Einreihen der Vielzahl von Nachrichten, die an den Benutzer übermittelt werden sollen, in der gereihten Reihenfolge in eine Warteschlange; und
Konvertieren der Warteschlange der Vielzahl von Nachrichten in ein Sprachformat;
und
einen oder mehrere Lautsprecher (210), die konfiguriert sind, um die konvertierte Vielzahl von Nachrichten an den Benutzer zu übermitteln.

2. Drahtloses Kommunikationssystem (100) nach Anspruch 1, wobei die mindestens eine persönliche Schutzausrüstungsvorrichtung (120) eine Vielzahl von persönlichen Schutzausrüstungsvorrichtungen (120) umfasst, und wobei jede der Vielzahl von persönlichen Schutzausrüstungsvorrichtungen (120) mit einer Prioritätsstufe assoziiert ist.

3. Drahtloses Kommunikationssystem (100) nach Anspruch 2, wobei der mindestens eine Prozessor (326) ferner konfiguriert ist zum:
Reihen der Vielzahl von Nachrichten basierend auf der Prioritätsstufe der mit den Nachrichten assoziierten persönlichen Schutzausrüstungsvorrichtungen (120).

4. Drahtloses Kommunikationssystem (100) nach Anspruch 1, ferner umfassend ein drahtloses Kommunikationsmodul (320) des Headsets (110), wobei das drahtlose Kommunikationsmodul (320) ein Nahfeldkommunikationsmodul umfasst, und wobei jede der mindestens einen persönlichen Schutzausrüstungsvorrichtungen (120) ein Nahfeldkommunikationsmodul umfasst.

5. Drahtloses Kommunikationssystem (100) nach Anspruch 4, wobei, wenn sich die persönliche Schutzausrüstungsvorrichtung (120) in der Nähe des Headsets (110) innerhalb des Nahfeldkommunikationsbereichs befindet, der mindestens eine Prozessor (326) konfiguriert ist zum:
Durchführen eines Peer-to-Peer-Datenaustauschs mittels Nahfeldkommunikation zwischen dem Headset (110) und der persönlichen Schutzausrüstungsvorrichtung (120); und
automatischen Aktivieren einer Bluetooth-Verbindung zwischen dem Headset (110) und der persönlichen Schutzausrüstungsvorrichtung (120) über den Peer-to-Peer-Datenaustausch.

6. Drahtloses Kommunikationssystem (100) nach einem der Ansprüche 1 bis 5, wobei das drahtlose Kommunikationsmodul (320) des Headsets (110) ein Bluetooth-Kommunikationsmodul und einen Text-zu-Sprache-Konverter umfasst.

7. Verfahren zur Übermittlung von Daten von einer persönlichen Schutzausrüstungsvorrichtung (120) an ein Headset (110), umfassend:
Erzeugen einer Vielzahl von Nachrichten durch eine persönliche Schutzausrüstungsvorrichtung (120), umfassend Informationen, die sich auf die persönliche Schutzausrüstungsvorrichtung (120) beziehen;
Bestimmen einer Prioritätsstufe für die Vielzahl von erzeugten Nachrichten basierend auf einer voreingestellten Liste von Prioritätsstufen, wobei die voreingestellte Liste von Prioritätsstufen für eine Vielzahl von persönlichen Schutzausrüstungsvorrichtung(en) (120) gleich ist;
drahtloses Übermitteln der Vielzahl von erzeugten Nachrichten von der persönlichen Schutzausrüstungsvorrichtung (120);
Empfangen einer Vielzahl von Nachrichten von einer oder mehreren persönlichen Schutzausrüstungsvorrichtungen (120) durch das Headset mit Gehörschutz (110);
Reihen der Vielzahl von Nachrichten durch das Headset mit Gehörschutz (110) basierend auf der bestimmten Prioritätsstufe; und
Einreihen der Vielzahl von Nachrichten, die an den Benutzer übermittelt werden sollen, durch das Headset mit Gehörschutz (110) in der gereihten Reihenfolge in eine Warteschlange,
Konvertieren der Warteschlange der Vielzahl von Nachrichten durch das Headset mit Gehörschutz (110) in ein Sprachformat; und
Übermitteln der Vielzahl von konvertierten Nachrichten über einen oder mehrere Lautsprecher (210) innerhalb des Headsets mit Gehörschutz (110) an den Benutzer.

8. Verfahren nach Anspruch 7, wobei das Bestimmen der Prioritätsstufe von der persönlichen Schutzausrüstungsvorrichtung (120) durchgeführt wird.

9. Verfahren nach einem der Ansprüche 7 oder 8, ferner umfassend:
Bestimmen einer Prioritätsstufe für jede der Vielzahl von persönlichen Schutzausrüstungsvorrichtungen (120);
Reihen der Vielzahl von Nachrichten basierend auf der bestimmten Prioritätsstufe der mit den Nachrichten assoziierten persönlichen Schutzausrüstungsvorrichtungen (120).

10. Verfahren nach einem der Ansprüche 7 bis 9, ferner umfassend ein Schützen eines Benutzers vor schädlicher Schallbelastung durch Material zur passiven Geräuschunterdrückung (112) innerhalb des Headsets mit Gehörschutz (110).

11. Verfahren nach einem der Ansprüche 7 bis 10, ferner umfassend:
Platzieren der persönlichen Schutzausrüstungsvorrichtung (120) in der Nähe des Headsets (110) innerhalb des Nahfeldkommunikationsbereichs;
Durchführen eines Peer-to-Peer-Datenaustauschs mittels Nahfeldkommunikation zwischen dem Headset (110) und der persönlichen Schutzausrüstungsvorrichtung (120); und
automatisches Aktivieren einer Bluetooth-Verbindung zwischen dem Headset (110) und der persönlichen Schutzausrüstungsvorrichtung (120) über den Peer-to-Peer-Datenaustausch.

12. Verfahren nach Anspruch 11, wobei das drahtlose Übermitteln der Vielzahl von erzeugten Nachrichten von der persönlichen Schutzausrüstungsvorrichtung (120) und das Empfangen der Vielzahl von erzeugten Nachrichten durch das Headset mit Gehörschutz (110) über Bluetooth durchgeführt wird.

13. Drahtloses Kommunikationssystem (100) nach Anspruch 1, wobei die Prioritätsstufe eine erste Kritikalitätsstufe und eine zweite Kritikalitätsstufe umfasst, und
wobei die erste Kritikalitätsstufe mindestens einem von Gasdetektionsalarmen, Temperaturstufenalarmen, Alarmen auf Atmungsebene entspricht, und die zweite Kritikalitätsstufe mindestens einem von Batteriestandalarmen, Sensoralarmen hinsichtlich niedrigeren Füllstands und Wartungsalarmen entspricht.

## Revendications

1. Système de communication sans fil (100) comprenant :
au moins un dispositif d'équipement de protection individuelle (120), configuré pour :
générer une pluralité de messages et pour communiquer sans fil la pluralité de messages générés ; et
déterminer un niveau de priorité pour la pluralité de messages générés en fonction d'une liste prédéfinie de niveaux de priorité ;
un casque de protection auditive (110) comprenant :
un matériau d'annulation passive de bruit (112) conçu pour protéger un utilisateur contre une exposition sonore nuisible ;
au moins un processeur (326) configuré pour :
recevoir une pluralité de messages d'au moins un dispositif d'équipement de protection individuelle (120) ;
classer la pluralité de messages en fonction du niveau de priorité déterminé ; et
mettre en file d'attente, dans l'ordre de classement, la pluralité de messages à communiquer à l'utilisateur ; et
convertir la file d'attente de la pluralité de messages au format vocal ; et
un ou plusieurs haut-parleurs (210) conçus pour communiquer la pluralité convertie de messages à l'utilisateur.

2. Système de communication sans fil (100) selon la revendication 1, dans lequel l'au moins un dispositif d'équipement de protection individuelle (120) comprend une pluralité de dispositifs d'équipement de protection individuelle (120) et dans lequel chaque dispositif de la pluralité de dispositifs d'équipement de protection individuelle (120) est associé à un niveau de priorité.

3. Système de communication sans fil (100) selon la revendication 2, dans lequel l'au moins un processeur (326) est en outre configuré pour :
classer la pluralité de messages en fonction du niveau de priorité des dispositifs d'équipement de protection individuelle (120) associés aux messages.

4. Système de communication sans fil (100) selon la revendication 1, comprenant en outre un module de communication sans fil (320) du casque (110), le module de communication sans fil (320) comprenant un module de communication en champ proche, et chacun des au moins un dispositif d'équipement de protection individuelle (120) comprenant un module de communication en champ proche.

5. Système de communication sans fil (100) selon la revendication 4, dans lequel, lorsque le dispositif d'équipement de protection individuelle (120) est à proximité du casque (110) dans une plage de communication en champ proche, l'au moins un processeur (326) est configuré pour :
achever un échange de données poste-à-poste en communication en champ proche entre le casque (110) et le dispositif d'équipement de protection individuelle (120) ; et
activer automatiquement une connexion Bluetooth entre le casque (110) et le dispositif d'équipement de protection individuelle (120) par le biais de l'échange de données poste-à-poste.

6. Système de communication sans fil (100) selon l'une quelconque des revendications 1 à 5, dans lequel le module de communication sans fil (320) du casque (110) comprend un module de communication Bluetooth ; et un convertisseur texte-parole.

7. Procédé de communication de données d'un dispositif d'équipement de protection individuelle (120) à un casque (110) comprenant :
la génération d'une pluralité de messages, par un dispositif d'équipement de protection individuelle (120), comprenant des informations relatives au dispositif d'équipement de protection individuelle (120) ;
la détermination d'un niveau de priorité pour la pluralité de messages générés en fonction d'une liste prédéfinie de niveaux de priorité, la liste prédéfinie de niveaux de priorité étant commune sur l'ensemble d'une pluralité de dispositifs d'équipement de protection individuelle (120) ;
la communication sans fil de la pluralité de messages générés à partir du dispositif d'équipement de protection individuelle (120) ;
la réception, par le casque de protection auditive (110), d'une pluralité de messages provenant d'un ou de plusieurs dispositifs d'équipement de protection individuelle (120) ;
le classement, par le casque de protection auditive (110), de la pluralité de messages en fonction du niveau de priorité déterminé ; et
la mise en file d'attente, dans l'ordre de classement, de la pluralité de messages à communiquer à l'utilisateur par le casque de protection auditive (110),
la conversion, par le casque de protection auditive (110), de la file d'attente de la pluralité de messages au format vocal ; et
la communication, par le biais d'un ou de plusieurs haut-parleurs (210) à l'intérieur du casque de protection auditive (110), de la pluralité de messages convertis à l'utilisateur.

8. Procédé selon la revendication 7, dans lequel la détermination du niveau de priorité est effectuée par le dispositif d'équipement de protection individuelle (120).

9. Procédé selon l'une quelconque des revendications 7 ou 8, comprenant en outre :
la détermination d'un niveau de priorité pour chacun des dispositifs de la pluralité de dispositifs d'équipement de protection individuelle (120) ;
le classement de la pluralité de messages en fonction du niveau de priorité déterminé des dispositifs d'équipement de protection individuelle (120) associés aux messages ; et

10. Procédé selon l'une quelconque des revendications 7 à 9, comprenant en outre la protection d'un utilisateur contre une exposition sonore nuisible par le biais d'un matériau d'annulation passive de bruit (112) à l'intérieur du casque de protection auditive (110).

11. Procédé selon l'une quelconque des revendications 7 à 10, comprenant en outre :
le placement du dispositif d'équipement de protection individuelle (120) à proximité du casque (110) dans une plage de communication en champ proche ;
l'achèvement d'un échange de données de communication poste-à-poste en champ proche entre le casque (110) et le dispositif d'équipement de protection individuelle (120) ; et
l'activation automatique d'une connexion Bluetooth entre le casque (110) et le dispositif d'équipement de protection individuelle (120) par le biais de l'échange de données poste-à-poste.

12. Procédé selon la revendication 11, dans lequel la communication sans fil de la pluralité de messages générés à partir du dispositif d'équipement de protection individuelle (120) et la réception de la pluralité de messages générés par le casque de protection auditive (110) sont effectuées via Bluetooth.

13. Système de communication sans fil (100) selon la revendication 1, dans lequel le niveau de priorité comprend un premier niveau de criticité et un second niveau de criticité, et
le premier niveau de criticité correspondant à des alarmes de détection de gaz ou des alarmes de niveau de température ou des alarmes de niveau respiratoire, et le second niveau de criticité correspondant à des alertes de niveau de batterie ou des alertes de capteur de niveau inférieur ou des alertes de maintenance.
